# EUROPÄISCHE PATENTANMELDUNG

(11) **EP 4 700 554 A1**
(43) Veröffentlichungstag der Anmeldung: **25.02.2026**
(21) Anmeldenummer: 25195836.9
(22) Anmeldetag: 14.08.2025
(51) Int. Cl.: G06F 3/04842, A61B 17/00, G06F 3/00, G06F 3/048, A61B 3/09, A61B 3/113, G06F 3/01, A61B 34/00, G02B 27/00

(54) **VORRICHTUNG UND VERFAHREN ZUM ERZEUGEN EINES STEUERSIGNALS FÜR EIN MEDIZINISCHES INSTRUMENT ODER EINE MEDIZINISCHE BILDGEBUNGSEINRICHTUNG**

(30) Priorität: 19.08.2024 DE 102024123657
(71) Anmelder: Karl Storz SE & Co. KG, 78532 Tuttlingen (DE)
(72) Erfinder: BRÜDERLE, Klaus, 78532 Tuttlingen (DE)

(57) **Zusammenfassung**

Die Erfindung schafft eine Vorrichtung und ein Verfahren zum Erzeugen eines Steuersignals für ein medizinisches Instrument und/oder eine medizinische Bildgebungseinrichtung. Die Vorrichtung umfasst:
eine Akkommodations-Erfassungseinrichtung (110), welche dazu eingerichtet ist, einen Akkommodationszustand (2) eines menschlichen Auges (1) zu erfassen; und
eine Ausgabeeinrichtung (120), welche dazu eingerichtet ist, basierend auf dem erfassten Akkommodationszustand (2) ein Steuersignal (79) für das medizinische Instrument (200) und/oder die medizinische Bildgebungseinrichtung (300) zu erzeugen und auszugeben.

## Beschreibung

### Technisches Gebiet der Erfindung

Die vorliegende Erfindung betrifft eine Vorrichtung und ein Verfahren zum Erzeugen eines Steuersignals für ein medizinisches Instrument und/oder eine medizinische Bildgebungseinrichtung.

### Hintergrund der Erfindung

In der modernen Medizin finden bildgebende Verfahren häufige Anwendung. Dabei wird eine medizinische Bildgebungseinrichtung verwendet, um Bilder einer medizinischen Szenerie zu erfassen. Diese Bilder können einem Benutzer, zum Beispiel einem Operateur, Informationen bereitstellen, die der Benutzer ohne Hilfsmittel visuell nicht erfassen könnte, sei es, weil die Bilder von einem nicht direkt sichtbaren Ort stammen (z.B. aus dem Inneren eines Patienten), oder sei es, weil die Informationen auf elektromagnetischer Strahlung mit nicht-sichtbaren Wellenlängen basieren.

Häufig kann die Bildgebungseinrichtung dabei gesteuert werden, um verschiedene Bereiche anzuzeigen, Vergrößerungen, durchzuführen und dergleichen mehr. Hierzu benötigt es einer Benutzerschnittstelle, mittels welcher ein Benutzer Steuersignale an die Bildgebungseinrichtung übermitteln kann.

Bei Bildgebungseinrichtungen, welche mehr nicht nur 2-dimensionale, sondern 3-dimensionale (oder noch höherdimensionale) Bildinhalte darstellen, ist es vorteilhaft, wenn die Benutzerschnittstelle dazu in der Lage ist, Steuersignale nicht nur bezüglich 2 Dimensionen (z.B. links/rechts und hoch/runter wie bei einem typischen Bildschirm), sondern zusätzlich auch noch bezüglich einer Tiefendimension (nach vorn/nach hinten) auszugeben.

Aus dem Bereich der 3D-Navigation sind CAD-Systeme bekannt, für welche spezielle 3D-Mäuse verwendet werden. Diese erfordern einerseits beträchtliche Übung für die richtige Handhabung, anderseits müssen diese für den Einsatz im medizinischen oder klinischen Umfeld aufwändig präpariert werden, beispielsweise steril verpackt werden. Darüber hinaus wird gerade für die Steuerung von medizinischen Instrumenten und medizinischen Bildgebungseinrichtungen eine möglichst intuitive und komfortable Steuerung bevorzugt.

### Zusammenfassung der Erfindung

Es ist daher eine Aufgabe der vorliegenden Erfindung, eine verbesserte Vorrichtung sowie ein verbessertes Verfahren zum Erzeugen eines Steuersignals für ein medizinisches Instrument und/oder eine medizinische Bildgebungseinrichtung bereitzustellen.

Diese Aufgaben werden durch die Gegenstände der unabhängigen Patentansprüche gelöst.

Gemäß einem ersten Aspekt stellt die Erfindung eine Vorrichtung zum Erzeugen eines Steuersignals für ein medizinisches Instrument und/oder eine medizinische Bildgebungseinrichtung, umfassend:
eine Akkommodations-Erfassungseinrichtung, welche dazu eingerichtet ist, einen Akkommodationszustand eines menschlichen Auges zu erfassen; und
eine Ausgabeeinrichtung, welche dazu eingerichtet ist, basierend auf dem erfassten Akkommodationszustand ein Steuersignal für das medizinische Instrument und/oder die medizinische Bildgebungseinrichtung zu erzeugen und auszugeben.

Unter der "Akkommodation" wird generell eine dynamische Anpassung der Brechkraft des menschlichen Auges verstanden. Durch Akkommodation können Objekte in verschiedenen Entfernungen für das menschliche Sehen scharf gestellt werden. Unter einem Akkommodationszustand ist somit ein Zustand des menschlichen Auges zu verstehen, welcher durch Akkommodation veränderbar ist, oder ein Ergebnis einer Akkommodation darstellt. Der Akkommodationszustand kann beispielsweise einen Krümmungszustand einer Linse des menschlichen Auges, eine absolute oder relative Dicke der Linse, eine Brechkraft der Linse und/oder dergleichen sein oder umfassen.

Durch die Verwendung des Akkommodationszustands als Basis für Steuersignale können diese Steuersignale somit insbesondere ohne Zuhilfenahme der Hände oder anderer Gliedmaßen erfolgen. Ein Benutzer eines medizinischen Instruments und/oder einer medizinischen Bildgebungseinrichtung hat daher, unter Verwendung der vorliegenden Erfindung, beide Hände frei, während er die Steuersignale erzeugt.

Darüber hinaus ermöglicht die vorliegende Lösung eine objektiv verbesserte Mensch-Maschine-Interaktion, da es für jeden Menschen - rein physiologisch - natürlich ist, die Tiefendimension mit der Akkommodation des Auges in Verbindung zu bringen, etwa bei dem Versuch, weit entfernte Objekte mit dem bloßen Auge scharf zu erfassen. Diese natürliche und intuitive Reaktion wird für die vorliegende Erfindung ausgenutzt.

Gemäß einigen bevorzugten Ausführungsformen, Varianten oder Verfeinerungen von Ausführungsformen ist die Ausgabeeinrichtung dazu eingerichtet, das Steuersignal derart zu erzeugen, dass es Positionen, Ausrichtungen, und/oder Bewegungen in drei orthogonalen Raumrichtungen angibt, also beispielsweise in und/oder nach: links/recht, oben/unten und vorne/hinten. Dabei kann es vorgesehen sein, dass einstellbar ist, ob das aktuell zu erzeugende Steuersignal eine Position angeben soll, etwa um in einem 3D-Bild einen bestimmten Punkt in einem echten oder virtuellen 3-dimensionalen Raum zu markieren, oder ob das Steuersignal eine Ausrichtung angeben soll, etwa um einen Blickwinkel einer medizinischen Bildgebungseinrichtung wie z.B. einer Kamera zu schwenken, und/oder ob das Steuersignal eine Bewegung angeben soll, etwa um eine medizinische Sonde, Drohne, Kamera etc. zu steuern.

Insbesondere basiert bevorzugt die Position, Ausrichtung, und/oder Bewegung bezüglich einer ersten dieser drei orthogonalen Raumrichtungen, und zwar besonders bevorzugt einer Tiefendimension, auf dem erfassten Akkommodationszustand. Wie bereits erwähnt wurde, ist dies eine besonders intuitive Verknüpfung, die dem Benutzer die Steuerung objektiv erleichtert.

Mit anderen Worten soll der Akkommodationszustand mit der Position, Ausrichtung und/oder Bewegung in der ersten bzw. in die erste Raumrichtung korrelieren. Die Korrelation kann proportional ausgestaltet sein, oder auch polynomial (mit Polynomen zweiten oder höheren Grades) oder exponentiell, oder gemäß einer benutzerdefinierten Funktion ausgestaltet sein. Hierdurch kann beispielsweise dem Umstand Rechnung getragen werden, dass bei Positionsbestimmungen oder Bewegungen in kurzen Distanzen oft hohe Präzision gefordert ist, während bei Positionsbestimmungen oder Bewegungen über größere Distanzen hinweg eine schnelle Veränderung/Bewegung bevorzugt wird.

Unter der Tiefendimension ist eine solche Dimension zu verstehen, entlang welcher sich Objekte in direkter Linie auf den Betrachter zu oder von dem Betrachter weg bewegen, ohne dass sie sich dabei nach links oder nach rechts, nach oben oder nach unten bewegen. Unter dem "Betrachter" kann hier sowohl der Nutzer der Vorrichtung verstanden werden, welcher einen realen oder virtuellen 3-dimensionalen Raum wahrnimmt, oder ein Objektiv einer Bildgebungseinrichtung, welche Bilddaten eines realen 3-dimensionalen Raums erfasst.

Alternativ kann auch vorgesehen sein, dass die Vorrichtung nur Steuersignale bezüglich einer oder zwei orthogonalen Raumrichtungen ausgibt, von denen eine, wiederum bevorzugt die Tiefendimension, mit dem erfassten Akkommodationszustand des menschlichen Auges korreliert.

Gemäß einigen bevorzugten Ausführungsformen, Varianten oder Verfeinerungen von Ausführungsformen kann die Vorrichtung außerdem eine Orientierungs-Erfassungseinrichtung, umfassen welche dazu eingerichtet ist, eine Orientierung einer Pupille eines menschlichen Auges zu erfassen, wobei Positionen und/oder Bewegungen in einer zweiten sowie einer dritten der drei orthogonalen Raumrichtungen auf der erfassten Orientierung basieren.

Beispielsweise kann anhand der Orientierung der Pupille eine Position oder Bewegung in den Richtungen links/rechts und oben/unten bestimmt oder gesteuert werden, während durch den erfassten Akkommodationszustand eine Position, Ausrichtung, oder Bewegung in der Richtung nach vorne/nach hinten, also in der Tiefendimension, bestimmt oder gesteuert wird. Mit anderen Worten kann die Orientierung der Pupille erfasst werden und darauf basierend eine Cursorsteuerung implementiert werden.

Somit ergibt sich erkennbar eine besonders intuitive Steuerung von medizinischen Instrumenten oder medizinischen Bildgebungseinrichtungen, bei welcher ein Mensch sein Steuerinteresse lediglich auf dieselbe Weise kundzutun braucht, wie er dies auch tun würde, um ein interessierendes Objekt näher zu betrachten: durch Bewegung der Pupille nach links/rechts/oben/unten und durch Akkommodation seines Auges, d.h., durch Verändern des Akkommodationszustands. Mit anderen Worten kann in dem Steuersignal eine Angabe vorne/hinten basierend auf der Fokussierung (oder: Weitenschärfe) des Auges erzeugt werden, und eine Angabe links/rechts/oben/unten basierend auf der Orientierung (oder: Ausrichtung) des Auges.

Als Orientierungs-Erfassungseinrichtung kann beispielsweise eine Kamera verwendet werden (etwa die Kamera einer Datenbrille), in Verbindung mit im Stand der Technik bekannten Methoden zur Pupillen-Nachverfolgung (engl. "eye tracking").

Gemäß einigen bevorzugten Ausführungsformen, Varianten oder Verfeinerungen von Ausführungsformen weist die Akkommodations-Erfassungseinrichtung eine Strahlungsquelle, eine Strahlungs-Erfassungseinheit, und eine Recheneinheit auf.

Die Strahlungsquelle ist dazu eingerichtet, eine Mess-Strahlung in das menschliche Auge auszusenden. Die Strahlungs-Erfassungseinheit ist dazu eingerichtet, eine von dem menschlichen Auge zurückgeworfene, auf der ausgesendeten Mess-Strahlung basierende Antwort-Strahlung zu erfassen. Die Recheneinheit ist dazu ausgebildet, basierend auf der erfassten Antwort-Strahlung den Akkommodationszustand des menschlichen Auges zu bestimmen.

Die von der Strahlungsquelle erzeugte Mess-Strahlung tritt in das Auge ein, und wird - beeinflusst von dem aktuellen Akkommodationszustand des Auges, etwa der Krümmung der Linse - in unterschiedlicher Weise als die Antwort-Strahlung reflektiert. Somit kann durch die Strahlungs-Erfassungseinheit mindestens eine Eigenschaft der Antwort-Strahlung erfassen und darauf basierend den Akkommodationszustand bestimmen.

Gemäß einigen bevorzugten Ausführungsformen, Varianten oder Verfeinerungen von Ausführungsformen ist die Strahlungsquelle zum Aussenden der Mess-Strahlung mit einer Wellenlänge von 600 Nanometern bis 800 Nanometern, insbesondere von 625 Nanometern bis 700 Nanometern, besonders bevorzugt von 650 Nanometern bis 675 Nanometern, ausgebildet. Diese Wellenlängen haben sich als einerseits ausreichend für eine gute und präzise Erfassung, andererseits als gesundheitlich unschädlich und störungsfrei für Benutzer erwiesen.

Gemäß einigen bevorzugten Ausführungsformen, Varianten oder Verfeinerungen von Ausführungsformen weist die Strahlungsquelle zum Aussenden der Mess-Strahlung einen punktförmigen, linienförmigen oder kreuzförmigen Strahlungsemitter auf.

Eine besonders schmal emittierte (also insbesondere punktförmig emittierte) Mess-Strahlung hat den Vorteil, dass diese in einem besonders schmalen Kanal verläuft und somit deren Reflektion, also die auf ihr basierende Antwort-Strahlung, besonders einfach detektierbar ist. Zudem kann bei gleicher Empfindlichkeit der Strahlungs-Erfassungseinheit eine Strahlungsquelle mit geringerer Leistung verwendet werden.

Gemäß einigen bevorzugten Ausführungsformen, Varianten oder Verfeinerungen von Ausführungsformen weist die Vorrichtung eine Datenbrille auf. Zumindest die Strahlungsquelle und/oder die Strahlungs-Erfassungseinheit ist bevorzugt an der Datenbrille ausgebildet, besonders bevorzugt beide. Auf diese Weise braucht ein Benutzer lediglich die Datenbrille aufzusetzen und kann somit bereits die Steuersignale erzeugen, d.h., das medizinische Instrument und/oder die medizinische Bildgebungseinrichtung steuern, ohne die Hände oder auch nur die Füße dafür verwenden zu müssen. Die Hände bleiben somit frei um beispielsweise ein oder mehrere weitere Instrumente zu führen, zum Beispiel ein Skalpell, ein Endoskop mit einer Funktionseinheit wie etwa einem Maulteil, und dergleichen mehr.

Gemäß einigen bevorzugten Ausführungsformen, Varianten oder Verfeinerungen von Ausführungsformen ist die Strahlungsquelle dazu eingerichtet, die Mess-Strahlung exzentrisch in das menschliche Auge einzustrahlen. Dies verbessert die Erfassung des Akkommodationszustands, denn exzentrisch, also entfernt vom Zentrum des menschlichen Auges (d.h. Augapfels), ist der Akkommodationszustand besser erfassbar. Beispielsweise würde eine zentral in das Auge einfallende Mess-Strahlung im Wesentlichen senkrecht auf die Linse auftreffen und daher bei unterschiedlichen Krümmungen der Linse kaum unterschiedlich beeinflusst werden. Auf exzentrisch eintreffende Mess-Strahlung wirkt sich die Krümmung der Linse (als ein möglicher Parameter des Akkommodationszustands) entsprechend deutlich stärker aus.

Gemäß einigen bevorzugten Ausführungsformen, Varianten oder Verfeinerungen von Ausführungsformen ist die Strahlungsquelle und/oder die Strahlungs-Erfassungseinheit zur stationären Montage in einem Raum eingerichtet ist oder dort montiert. Bei dem Raum kann es sich um einen Raum handeln, in dem oder von dem aus das medizinische Instrument und/oder die medizinische Bildgebungseinrichtung gesteuert werden soll, beispielsweise einen Operationssaal.

Gemäß einigen bevorzugten Ausführungsformen, Varianten oder Verfeinerungen von Ausführungsformen ist das Steuersignal dazu ausgebildet ist, eine Bildselektion, eine Fokusselektion, und/oder eine Motivselektion in einer 3D-Darstellung einzustellen. Bei der 3D-Darstellung kann es sich insbesondere um eine 3D-Projektion handeln. Unter eine 3D-Projektion ist dabei die Projektion einer 3D-Darstellung in oder für das menschliche Auge zu verstehen, beispielsweise durch eine Datenbrille. Umfasst die vorliegende Erfindung eine Datenbrille, kann diese vorteilhaft zusätzlich zu anderen Elemente auch noch eine Projektionseinheit zum Projizieren der 3D-Projektion in das menschliche Auge umfassen. Die 3D-Projektion kann auch durch ein Head-Up-Display erfolgen, welches ebenfalls Teil der erfindungsgemäßen Vorrichtung sein kann.

Gemäß einigen bevorzugten Ausführungsformen, Varianten oder Verfeinerungen von Ausführungsformen ist das Steuersignal dazu ausgebildet, eine Positions- oder Objektselektion in einer Virtuelle-Realität-Umgebung oder einer Erweiterte-Realität-Umgebung durchzuführen. Die Virtuelle-Realität-Umgebung oder Erweiterte-Realität-Umgebung kann wiederum durch eine Datenbrille, insbesondere durch eine Datenbrille der erfindungsgemäßen Vorrichtung, einem Benutzer bereitgestellt werden.

Gemäß einem zweiten Aspekt stellt die Erfindung ein Verfahren zum Erzeugen eines Steuersignals für ein medizinisches Instrument und/oder eine medizinische Bildgebungseinrichtung bereit, umfassend:
Erfassen eines Akkommodationszustands eines menschlichen Auges; und
Erzeugen und Ausgeben eines Steuersignals für ein medizinisches Instrument oder eine medizinische Bildgebungseinrichtung basierend auf dem erfassten Akkommodationszustand.

Gemäß einigen bevorzugten Ausführungsformen, Varianten oder Verfeinerungen von Ausführungsformen umfasst das Erfassen des Akkommodationszustands zumindest die Schritte:
Erzeugen einer Mess-Strahlung;
Aussenden der Mess-Strahlung in das menschliche Auge;
Erfassen einer auf der ausgesendeten Mess-Strahlung basierenden, zurückgeworfenen Antwort-Strahlung; und
Bestimmen des Akkommodationszustands basierend auf der erfassten Antwort-Strahlung.

Gemäß einem dritten Aspekt stellt die Erfindung ein Computerprogrammprodukt bereit, das ausführbaren Programmcode umfasst, welcher dazu eingerichtet ist, wenn er von einer Recheneinheit ausgeführt wird, das Verfahren gemäß einer Ausführungsform des zweiten Aspekts der vorliegenden Erfindung durchzuführen.

Gemäß einem vierten Aspekt stellt die Erfindung ein nicht-flüchtiges, computerlesbares Datenspeichermedium bereit, das ausführbaren Programmcode umfasst, welcher dazu eingerichtet ist, wenn er von einer Recheneinheit ausgeführt wird, das Verfahren gemäß einer Ausführungsform des zweiten Aspekts der vorliegenden Erfindung durchzuführen oder zu steuern.

Das nicht-flüchtige, computerlesbare Datenspeichermedium kann jede Art von Computerspeicher, insbesondere Halbleiterspeicher, wie z.B. einen Festkörperspeicher, umfassen oder aus einem solchen bestehen. Das Datenträger kann auch eine CD, eine DVD, eine Blu-Ray-Disc, einen USB-Speicherstick oder dergleichen umfassen oder daraus bestehen.

Gemäß einem fünften Aspekt stellt die Erfindung einen Datenstrom bereit, der ausführbaren Programmcode umfasst oder zum Erzeugen von ausführbarem Programmcode konfiguriert ist, welcher dazu eingerichtet ist, wenn er von einer Recheneinheit ausgeführt wird, das Verfahren gemäß einer Ausführungsform des zweiten Aspekts der vorliegenden Erfindung durchzuführen oder zu steuern.

Gemäß einem weiteren Aspekt stellt die Erfindung eine Datenbrille bereit, welche eine Ausführungsform des ersten Aspekts der vorliegenden Erfindung umfasst.

Weitere vorteilhafte Varianten, Optionen, Ausführungsformen und Modifikationen ergeben sich aus den folgenden Figuren, der ausführlichen Beschreibung, sowie aus den Ansprüchen. Es versteht sich jedoch, dass die detaillierte Beschreibung und die spezifischen Beispiele zwar bevorzugte Ausführungsformen der Erfindung angeben, aber nur zur Veranschaulichung angeführt werden, da verschiedene Änderungen und Modifikationen innerhalb des Umfangs der Erfindung für den Fachmann ersichtlich sind.

### Kurzbeschreibung der Figuren

Einzelne Ausführungsformen der vorliegenden Offenbarung werden anhand der folgenden Abbildungen im Detail erläutert werden. Die Bestandteile in den Zeichnungen sind nicht notwendigerweise maßstabsgetreu, sondern dienen dazu, die Grundsätze der vorliegenden Erfindung zu veranschaulichen. Teile in den verschiedenen Abbildungen, die den gleichen Elementen oder Verfahrensschritten entsprechen, wurden in den Abbildungen mit den gleichen Bezugszeichen versehen. Die Nummerierung von Verfahrensschritten dient zunächst nur zu deren Unterscheidung und impliziert nicht zwingend eine entsprechende Reihenfolge, wobei es jedoch eine Variante darstellt, die Schritte in der Reihenfolge ihrer Nummerierung durchzuführen. Mehrere Schritte können auch überlappend oder gleichzeitig durchgeführt werden. Von den Figuren zeigen:
- Fig. 1: eine schematische Darstellung einer Vorrichtung gemäß einer Ausführungsform der vorliegenden Erfindung;
- Fig. 2: mögliche Details der Vorrichtung aus Fig. 1 gemäß einer Ausführungsform der vorliegenden Erfindung;
- Fig. 3: ein schematisches Flussdiagramm zur Erläuterung eines Verfahrens gemäß einer weiteren Ausführungsform der vorliegenden Erfindung;
- Fig. 4: eine schematische Blockdarstellung zur Erläuterung eines Computerprogrammprodukts gemäß noch einer weiteren Ausführungsform der vorliegenden Erfindung; und
- Fig. 5: eine schematische Blockdarstellung zur Erläuterung eines Datenspeichermediums gemäß noch einer weiteren Ausführungsform der vorliegenden Erfindung.

### Detaillierte Beschreibung der Figuren

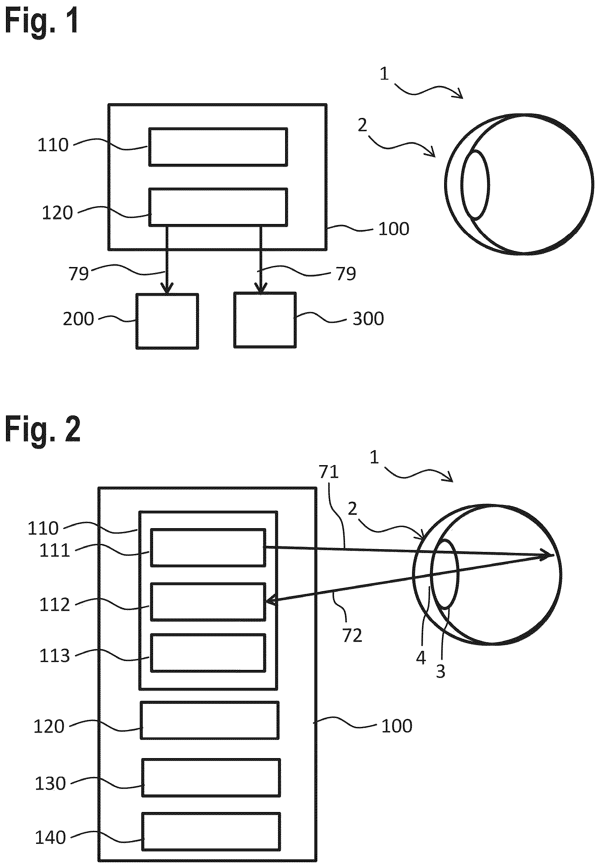

Fig. 1 zeigt eine schematische Darstellung einer Vorrichtung gemäß einer Ausführungsform der vorliegenden Erfindung, d.h. einer Vorrichtung 100 zum Erzeugen eines Steuersignals 79 für ein medizinisches Instrument 200 und/oder eine medizinische Bildgebungseinrichtung 300. Das medizinische Instrument 200 kann beispielsweise ein Operationsroboter oder ein steuerbares Kamerasystem sein. Das Steuersignal 79 kann bei dem medizinischen Instrument 200 oder der medizinischen Bildgebungseinrichtung 300 beispielsweise eine Ausrichtung, eine Position und/oder eine Bewegungsrichtung steuern. Die erfindungsgemäße Vorrichtung 100 kann das zu steuernde medizinische Instrument 200 und/oder die zu steuernde medizinische Bildgebungseinrichtung 300 umfassen.

Bei der medizinischen Bildgebungseinrichtung 300 kann es sich beispielsweise um ein OP-Kamerasystem handeln, welches Bildinhalte anzeigt und, vorteilhaft, vielfältig kombiniert. Inhalte können nebeneinander, übereinander, oder ineinander dargestellt werden. Motive sind teilweise dreidimensional erfasst (zum Beispiel bei OP-Kamerasystemen, welche Stereo-Kameras umfassen). Eine zusätzliche Mehrdimensionalität kann durch zusätzliche Kanäle auftreten. Beispielsweise kann ein erster Kanal ein Echtfarbenbild umfassen, ein zweiter Kanal ein Falschfarbenbild und/oder dergleichen mehr. Eine Dimension, in welcher oder für welche das Steuersignal 79 eine Steuerung bewirken kann, kann somit auch die Dimension der Kanalnummern sein.

Das OP-Kamerasystem kann eine Kamera, eine Kamerasteuerung zum Verarbeiten der durch die Kamera erfassten Bilder, und eine Anzeigeeinrichtung zum Darstellen der erfassten und/oder der verarbeiteten Bilder aufweisen. Die Kamera kann an einem Mikroskop oder einem verschiebbaren Gestell befestigt sein und/oder eine endoskopische Kamera sein. Bei der medizinischen Bildgebungseinrichtung 300 kann es sich somit insbesondere um eine endoskopische Bildgebungseinrichtung handeln.

Das Steuersignal 79 kann eine oder mehrere der folgenden Steuerungen durchführen: eine Bildselektion in einer 3D-Darstellung, eine Fokusselektion in einer 3D-Darstellung, und/oder eine Motivselektion in einer 3D-Darstellung, wobei es sich bei der 3D-Darstellung jeweils um eine 3D-Projektion, z.B. in das Auge, handeln kann. Alternativ oder zusätzlich kann das Steuersignal dazu ausgebildet sein, eine Positions- oder Objektselektion in einer Virtuelle-Realität-Umgebung oder einer Erweiterte-Realität-Umgebung durchzuführen.

Die Vorrichtung 100 umfasst eine Akkommodations-Erfassungseinrichtung 110, welche dazu eingerichtet ist, einen Akkommodationszustand 2 eines menschlichen Auges 1 zu erfassen, sowie eine Ausgabeeinrichtung 120, welche dazu eingerichtet ist, basierend auf dem erfassten Akkommodationszustand 2 das Steuersignal 79 für das medizinische Instrument 200 und/oder die medizinische Bildgebungseinrichtung 300 zu erzeugen und auszugeben.

Insbesondere kann die Ausgabeeinrichtung 120 dazu eingerichtet sein, das Steuersignal 79 derart zu erzeugen, dass es Positionen, Ausrichtungen, und/oder Bewegungen in ein bis drei orthogonalen Raumrichtungen angibt. Die Position, Ausrichtung, und/oder Bewegung bezüglich einer ersten dieser orthogonalen Raumrichtungen, insbesondere einer Tiefendimension, basiert vorteilhaft auf dem erfassten Akkommodationszustand 2.

Beispielsweise kann der Akkommodationszustand 2 einen Krümmungszustand einer Linse 3 des Auges 1 umfassen, oder daraus bestehen. Ein erster Akkommodationszustand 2, also in diesem Beispiel ein erster Krümmungszustand der Linse 3, kann als Nulllage festgelegt werden, sodass Akkommodationszustände 2 mit stärkerer Krümmung der Linse 3 als der erste Krümmungszustand mit Koordinaten der Tiefendimension entlang einer ersten Richtung (z.B. negative z-Richtung) assoziiert werden können, und Akkommodationszustände 2 mit geringerer Krümmung der Linse 3 als der erste Krümmungszustand mit Koordinaten der Tiefendimension entlang einer zweiten, der ersten entgegengerichteten Richtung (z.B. positiven z-Richtung), assoziiert werden können.

Die negative z-Richtung ist dabei typischerweise eine Richtung von dem Betrachter (Kamera-Objektiv, Datenbrille, Auge 1 etc.) fort, während die positive z-Richtung zum Betrachter hin zeigt. Hierbei wird ein orthogonales Dreibein angenommen, in welchem eine positive x-Richtung von links nach rechts zeigt, eine positive y-Richtung von unten nach oben zeigt, und schließlich die positive z-Richtung von hinten nach vorne, also auf den Betrachter des Dreibeins, zeigt.

Je nach Anwendung können diese assoziierten Koordinaten durch die Ausgabeeinrichtung 120 dann in ein entsprechendes Steuersignal 79 umgewandelt werden, welches etwa eine Position, Bewegung, oder Ausrichtung gemäß der entsprechenden Koordinate anzeigen oder steuern kann. Somit kann ein Benutzer durch Akkommodieren seiner Augen 1 für weiter reichende Fernsicht mithilfe der Vorrichtung 100 ein Steuersignal 79 erzeugen lassen, welches etwa eine medizinische Bildgebungseinrichtung 300 steuert, weiter entfernte Details näher darzustellen, beispielsweise durch Heranzoomen und/oder Bewegen ihrer Eingangsoptik. Als ein Beispiel kann die Koordinate der Tiefendimension eine Projektionsebene für eine Cursorsteuerung in einem 3D-Bild angeben, insbesondere eine medizinische Bildgebungseinrichtung 300 steuern, diese Projektionsebene auszuwählen, eine Schnittansicht durch diese Ebene darzustellen, oder dergleichen.

Fig. 2 zeigt eine schematische Darstellung der Vorrichtung 100 mit Details gemäß einer Ausführungsform.

Die Vorrichtung 100 umfasst in dieser Variante eine Orientierungs-Erfassungseinrichtung 130, welche dazu eingerichtet ist, eine Orientierung einer Pupille 4 des menschlichen Auges 1 zu erfassen, beispielsweise mittels im Stand der Technik bekannter "eye-tracking"-Verfahren. Vorteilhaft können durch das erzeugte Steuersignal 79 angegebene (oder: angezeigte) Positionen, Ausrichtungen, und/oder Bewegungen in einer zweiten sowie - optional - einer dritten der drei orthogonalen Raumrichtungen auf der erfassten Orientierung der Pupille 4 basieren. Bezogen auf das oben beschriebene orthogonale Dreibein x, y, z, können somit durch das Steuersignal 79 Positionen, Ausrichtungen, oder Bewegungen in x- Richtung und/oder y-Richtung basierend auf der Orientierung der Pupille 4 angezeigt oder gesteuert werden.

Somit kann ein Benutzer beispielsweise nach links sehen, um ein Steuersignal 79 erzeugen zu lassen, welches eine Bewegung eines Sichtkegels einer medizinischen Bildgebungseinrichtung 300 nach links bewirkt, und dabei die Linse 3 derart krümmen (durch eine bewusste oder unbewusste Akkommodation), sodass das Steuersignal 79 zusätzlich so erzeugt wird, dass der Sichtkegel nach vorne bewegt wird, oder dergleichen.

Im Folgenden werden beispielhafte Details der Akkommodations-Erfassungseinrichtung 110 beschrieben. Diese können zusammen mit der Orientierungs-Erfassungseinrichtung 130 vorgesehen werden, aber auch separat davon. Mit anderen Worten kann die Akkommodations-Erfassungseinrichtung 110 mit den im Folgenden beschriebenen Details auch in einer Vorrichtung 100 vorgesehen sein, welche das Steuersignal 79 derart erzeugt, dass es lediglich Positionen, Ausrichtungen, oder Bewegungen in einer einzelnen Richtung/Dimension anzeigt oder angibt, insbesondere der Tiefendimension.

Die Akkommodations-Erfassungseinrichtung 110 kann insbesondere eine Strahlungsquelle 111, eine Strahlungs-Erfassungseinheit 112, und eine Recheneinheit 113 aufweisen.

Hierbei ist die Strahlungsquelle 111 dazu eingerichtet, eine Mess-Strahlung 71 zu erzeugen und in das menschliche Auge 1 auszusenden, und zwar beispielsweise mit einer Wellenlänge von 600 Nanometern bis 800 Nanometern, insbesondere von 625 Nanometern bis 700 Nanometern, besonders bevorzugt von 650 Nanometern bis 675 Nanometern. Wie in im Vorangehenden bereits erläutert wurde, eignen sich diese Wellenlängen besonders, um vom menschlichen Auge gut reflektiert zu werden, ohne störend zu wirken oder gar Schaden zu verursachen.

Die Strahlungsquelle 111 kann einen punktförmigen oder linienförmigen Emitter aufweisen, welcher an einer Datenbrille angeordnet sein kann.

Die Strahlungs-Erfassungseinheit 112 ist dazu eingerichtet, eine von dem menschlichen Auge 1 zurückgeworfene, auf der ausgesendeten Mess-Strahlung 71 basierende Antwort-Strahlung 72 zu erfassen. Eine solche Antwort-Strahlung 72 wird beispielsweise durch an der Retina (Netzhaut) reflektierte Mess-Strahlung 71 erzeugt. Die oben genannten Wellenlängen eignen sich hierfür besonders gut. Sind die Strahlungsquelle 111 und die Strahlungs-Erfassungseinheit 112 beide an einer Datenbrille angeordnet, insbesondere in die Datenbrille integriert, können sie vorteilhaft so zueinander angeordnet sein, dass zumindest ein Teil (bevorzugt der Großteil) der von dem menschlichen Auge 1 zurückgeworfenen Mess-Strahlung 71 als Antwort-Strahlung 72 auf die Strahlungs-Erfassungseinheit 112 auftrifft.

Die Strahlungs-Erfassungseinheit 112 kann beispielsweise als ein Halbleiter-Photosensor oder dergleichen ausgebildet sein.

Vorteilhaft entsendet die Strahlungsquelle 111 die Mess-Strahlung 71 exzentrisch in das menschliche Auge 1. Alternativ oder zusätzlich können die Strahlungsquelle 111 und die Strahlungs-Erfassungseinheit 112 derart angeordnet sein (insbesondere bezüglich einer Datenbrille, an der sie angeordnet sind), dass die Antwort-Strahlung 72 das menschliche Auge exzentrisch verlässt. Wie im Vorangehenden erläutert wurde, ist beispielsweise die Krümmung der Linse 3 an deren radialen Rändern deutlicher ausgeprägt als in ihrem radialen Zentrum. Deswegen ist die Mess-Strahlung 71 oder die Antwort-Strahlung 72, wenn sie die Linse 3 jeweils exzentrisch, also näher an deren radialen Rändern als an deren radialem Zentrum durchqueren, stärkeren Veränderungen (z.B. stärkerer Brechung) unterworfen, als bei einem zentrierten Durchqueren der Linse 3. Dementsprechend enthält die Antwort-Strahlung 72 bei exzentrischem Eindringen der Mess-Strahlung 71 und/oder bei exzentrischem Austreten der Antwort-Strahlung 72 *ceteris paribus* deutlicher ausgeprägte Informationen über den Akkommodationszustand 2, die dementsprechend mit weniger Aufwand, genauer, und akkurater bestimmt werden können.

Hierzu ist die Recheneinheit 113 vorgesehen. Diese ist dazu ausgebildet, basierend auf der erfassten Antwort-Strahlung 72 den Akkommodationszustand 2 des menschlichen Auges 1 zu bestimmen. In dem oben verwendeten Beispiel kann etwa durch Vergleich der (der Recheneinheit 113 bekannten) Richtung der Aussendung (oder sonstigen Eigenschaften) der Mess-Strahlung 71 mit der Einfallsrichtung (oder sonstigen Eigenschaften) der Antwort-Strahlung 72 durch die Recheneinheit 113 eine Brechung durch die Linse 3 ermittelt werden, welche wiederum einen Rückschluss auf die Krümmung der Linse 3 und somit deren Akkommodationszustand 2 ermöglicht.

Wie im Vorangehenden erläutert wurde, sind jedoch auch andere Varianten möglich, unterschiedliche Akkommodationszustände des Auges 1 zu bestimmen. Beispielsweise kann die Akkommodations-Erfassungseinrichtung 110 dazu eingerichtet sein, ein Bild des menschlichen Auges 1 aufzunehmen, und das aufgenommene Bild auszuwerten, um basierend auf den Bilddaten den Akkommodationszustand 2 zu bestimmen. Hierzu kann die Recheneinheit 113 beispielsweise ein Maschinenlernen-Modell (z.B. ein künstliches neuronales Netzwerk) implementieren, welches dazu trainiert ist, aus derartigen Bildern des menschlichen Auges 1 den Akkommodationszustand 2 des Auges 1 zu bestimmen.

Im Vorangehenden wurde vorrangig eine Ausführungsform beschrieben, in welcher zumindest die Strahlungsquelle 111 und die Strahlungs-Erfassungseinheit 112 (und optional zusätzlich die Recheneinheit 113) an einer Datenbrille angebracht oder in eine Datenbrille der Vorrichtung 100 integriert sind. In manchen Varianten kann sogar die gesamte Vorrichtung 100 in eine Datenbrille integriert sein. In anderen Varianten kann die Datenbrille über einen drahtlose oder drahtgebundenen Sender einer Kommunikationseinrichtung 140 der Vorrichtung 100 verfügen, mittels welcher sie mit anderen Bestandteilen der Vorrichtung 100, beispielsweise der Recheneinheit 113, kommuniziert.

Bei der Recheneinheit 113 kann es sich um jedwede Einrichtung handeln, die zum digitalen Rechnen, insbesondere zum Ausführen einer Software, einer Applikation, oder eines Algorithmus ausgebildet und eingerichtet ist. Die Recheneinheit 113 kann beispielsweise mindestens eine Prozessoreinheit (z.B. mindestens eine CPU), mindestens eine Graphikprozessoreinheit (z.B.: mindestens eine GPU), mindestens ein field-programmable gate array, FPGA und/oder mindestens eine applikationsspezifische integrierte Schaltung, ASIC, und/oder eine beliebige Kombination der vorgenannten Elemente umfassen. Die Recheneinheit 113 kann außerdem einen Arbeitsspeicher und/oder einen nichtflüchtigen Datenspeicher aufweisen, welche operativ miteinander und/oder mit einigen oder allen der zuvor genannten Elemente verknüpft sind. Die Recheneinheit 113 kann teilweise oder vollständig in einer lokalen Einheit (beispielsweise einem personal computer, PC, einem Laptop, einem Notebook oder dergleichen) und/oder teilweise oder vollständig in einem verteilten System implementiert sein.

Die Recheneinheit 113 kann beispielsweise durch einen Server und/oder eine Cloud-Computing-Plattform bereitgestellt werden. Die Recheneinheit 113 kann auch, zusammen mit der Ausgabeeinrichtung 120, in das zu steuernde medizinische Instrument 200 oder die zu steuernde medizinische Bildgebungseinrichtung 300 integriert sein. Auf diese Weise kann eine Datenbrille beispielsweise besonders leicht und bequem tragbar gehalten werden, indem diese etwa nur die Strahlungsquelle 111, die Strahlungs-Erfassungseinheit 112 sowie einen Teil (insbesondere einen Sender) der Kommunikationseinrichtung 140 zur Kommunikation mit den restlichen Bestandteilen der Vorrichtung 100 aufweist. Die Verarbeitung der Ausgangssignale der Strahlungs-Erfassungseinheit 112 kann dann beispielsweise erst nach deren Übermittlung, mittels der Kommunikationseinrichtung 140, an die getrennt angeordnete Recheneinheit 113 erfolgen, welche dort mit der Ausgabeeinrichtung 120 verbunden ist.

Die Kommunikationseinrichtung 140 kann hierzu mindestens einen Sender an der Datenbrille sowie einen Empfänger seitens der Recheneinheit 113 aufweisen, vorteilhaft jeweils Sende-/Empfänger. Die Recheneinheit 113 kann einzelne Einrichtungen oder Einheiten der Vorrichtung 100 mit implementieren, beispielsweise die Ausgabeeinrichtung 120.

Es sind jedoch auch Ausführungsformen möglich, in welchen keine Datenbrille vorhanden ist, oder die Datenbrille weniger Elemente/Einrichtungen/Einheiten aufweist, oder andere. In manchen Ausführungsformen beispielsweise kann die Strahlungsquelle 111 fest in einem Raum montierbar oder montiert sein (z.B. in einem Operationssaal oder Untersuchungszimmer), während die Strahlungs-Erfassungseinheit 112 auf der Datenbrille oder einem anderen von einem Nutzer getragenen Objekt (engl. "wearable"), z.B. einem Stirnband, montiert oder angeordnet ist. Ein Emitter der Strahlungsquelle 111 kann in diesem Fall ein von einem Punkt verschiedenes geometrisches Muster aufweisen, beispielsweise eine Linienform, eine Gitterform, oder eine Kreuzform.

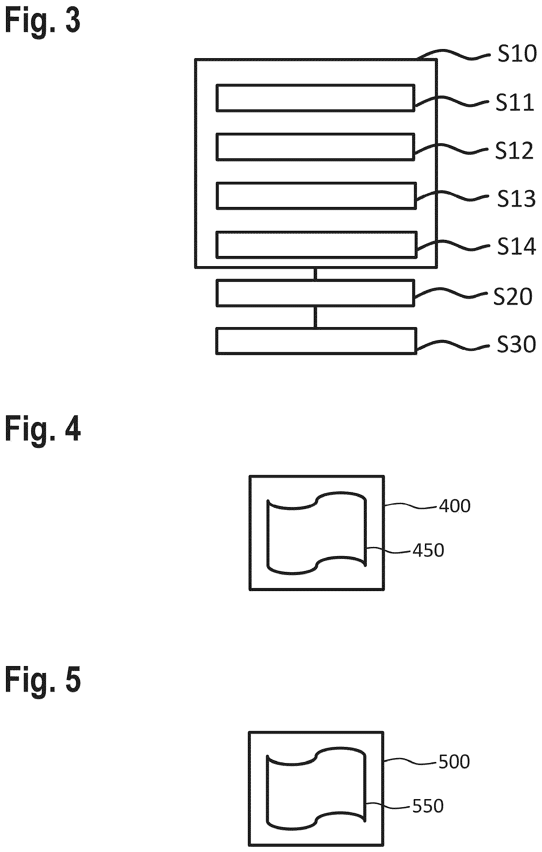

Fig. 3 zeigt ein schematisches Flussdiagramm zur Erläuterung eines Verfahrens gemäß einer Ausführungsform der vorliegenden Erfindung, d.h. eines Verfahrens zum Erzeugen eines Steuersignals für ein medizinisches Instrument 200 und/oder eine medizinische Bildgebungseinrichtung 300, insbesondere für eine 3D-Steuerung. Das Verfahren ist mit der erfindungsgemäßen Vorrichtung 100 ausführbar und ist gemäß allen mit Bezug auf die Vorrichtung 100 beschriebenen Varianten, Optionen, Ausführungsformen und Verfeinerungen von Ausführungsformen anpassbar und umgekehrt. Das Verfahren ist jedoch auch unabhängig von der Vorrichtung 100 durchführbar.

In einem ersten Schritt S10 des Verfahrens wird ein Akkommodationszustand 2 eines menschlichen Auges 1 erfasst, beispielsweise wie im Vorangehenden mit Bezug auf die Akkommodations-Erfassungseinrichtung 110 beschrieben wurde.

Dementsprechend kann das Erfassen S10 des Akkommodationszustands 2 beispielsweise die folgenden Teilschritte umfassen:
In einem optionalen Teilschritt S11 wird eine Mess-Strahlung 71 erzeugt, und in einem optionalen Teilschritt S12 wird die erzeugte Mess-Strahlung 71 in das menschliche Auge 1 ausgesendet, beispielsweise wie im Vorangehenden mit Bezug auf die Strahlungsquelle 111 beschrieben wurde.

Demnach kann die Mess-Strahlung 71 bevorzugt mit einer Wellenlänge von 600 Nanometern bis 800 Nanometern, insbesondere von 625 Nanometern bis 700 Nanometern, besonders bevorzugt von 650 Nanometern bis 675 Nanometern, erzeugt S11 werden.

In einem optionalen Teilschritt S13 wird eine auf der ausgesendeten Mess-Strahlung 71 basierende (z.B. von dieser erzeugte oder zumindest teilweise aus dieser bestehende), zurückgeworfene Antwort-Strahlung 72 erfasst, beispielsweise wie im Vorangehenden mit Bezug auf die Strahlungs-Erfassungseinheit 112 beschrieben wurde. In einem optionalen Teilschritt S14 wird der Akkommodationszustand 2 basierend auf der erfassten Antwort-Strahlung 72 bestimmt, beispielsweise wie im Vorangehenden mit Bezug auf die Recheneinheit 113 beschrieben wurde.

Das Aussenden S12 erfolgt bevorzugt exzentrisch bezüglich des menschlichen Auges 1, insbesondere derart, dass die Mess-Strahlung 71 und/oder die Antwort-Strahlung 72 eine Linse 3 des menschlichen Auges näher an radialen Rändern der Linse 3 durchquert bzw. durchqueren als an dem radialen Zentrum der Linse 3.

Wie im Vorangehenden bereits erläutert wurde, kann das Erfassen S10 des Akkommodationszustands 2 auch anders erfolgen, beispielsweise über eine Bilderfassung und eine Bildauswertung, insbesondere unter Verwendung eines Maschinenlernen-Modells.

In jedem Fall wird in einem Schritt S20 ein Steuersignal 79 für ein medizinisches Instrument 200 oder eine medizinische Bildgebungseinrichtung 300 basierend auf dem erfassten Akkommodationszustand 2 erzeugt und ausgegeben.

In einem Schritt S30 kann das medizinische Instrument 200 oder die medizinische Bildgebungseinrichtung 300 mittels des Steuersignals 79 gesteuert werden, beispielsweise zu einer der vielen im Vorangehenden beschriebenen Anwendungen.

Fig. 4 zeigt ein schematisches Blockdiagramm eines Computerprogrammprodukts 400 gemäß einer Ausführungsform des dritten Aspekts der vorliegenden Erfindung. Das Computerprogrammprodukt 400 umfasst ausführbaren Programmcode 450, welcher dazu eingerichtet ist, wenn er ausgeführt wird (z.B. durch eine Recheneinheit 113), das Verfahren gemäß einer Ausführungsform der vorliegenden Erfindung durchzuführen oder zu steuern, beispielsweise gemäß Fig. 3.

Fig. 5 zeigt ein schematisches Blockdiagramm eines nicht-flüchtigen computerlesbaren Datenspeichermediums 500 gemäß einer Ausführungsform der vorliegenden Erfindung. Das Datenspeichermedium 500 umfasst ausführbaren Programmcode 550, welcher dazu eingerichtet ist, wenn er ausgeführt wird (z.B. durch eine Recheneinheit 113), das Verfahren gemäß einer Ausführungsform der vorliegenden Erfindung durchzuführen oder zu steuern, beispielsweise gemäß Fig. 3.

Das nicht-flüchtige computerlesbare Datenspeichermedium 500 kann beispielsweise als ein Halbleiterspeicher, z. B. ein SSD-Speicherstein ausgebildet sein oder einen solchen aufweisen. Das Datenspeichermedium 500 kann auch eine CD, DVD, Blu-Ray oder eine magnetische Speichervorrichtung aufweisen oder umfassen.

Die vorstehende Beschreibung der offenbarten Ausführungsformen enthält lediglich Beispiele für mögliche Umsetzungen, die beschrieben werden, um einen Fachmann in die Lage zu versetzen, die vorliegende Erfindung herzustellen oder verwenden zu können. Verschiedene Variationen und Modifikationen dieser Ausführungsformen sind für den Fachmann - nach Kenntnis der vorliegenden Erfindung - leicht ersichtlich, und die hierin definierten allgemeinen Prinzipien können auf andere Ausführungsformen angewendet werden, ohne vom Umfang der vorliegenden Offenbarung abzuweichen.

Somit soll die vorliegende Erfindung nicht auf die hierin gezeigten, spezifischen Ausführungsformen beschränkt sein, sondern ihr soll der breiteste Umfang zugestanden werden, der mit den hierin offenbarten Prinzipien und Merkmalen übereinstimmt.

### Bezugszeichenliste

- 1: menschliches Auge
- 2: Akkommodationszustand
- 3: Linse
- 4: Pupille

- 71: Mess-Strahlung
- 72: Antwort-Strahlung
- 79: Steuersignal
- 100: Vorrichtung
- 110: Akkommodations-Erfassungseinrichtung
- 111: Strahlungsquelle
- 112: Strahlungs-Erfassungseinheit
- 113: Recheneinheit
- 120: Ausgabeeinrichtung
- 130: Orientierungs-Erfassungseinrichtung
- 140: Kommunikationseinrichtung
- 200: medizinisches Instrument
- 300: medizinische Bildgebungseinrichtung
- 400: Computerprogramprodukt
- 450: Programmcode
- 500: Datenspeichermedium
- 550: Programmcode

- S10..S30: Verfahrensschritte

## Patentansprüche

1. Vorrichtung (100) zum Erzeugen eines Steuersignals (79) für ein medizinisches Instrument (200) und/oder eine medizinische Bildgebungseinrichtung (300), umfassend:
eine Akkommodations-Erfassungseinrichtung (110), welche dazu eingerichtet ist, einen Akkommodationszustand (2) eines menschlichen Auges (1) zu erfassen; und
eine Ausgabeeinrichtung (120), welche dazu eingerichtet ist, basierend auf dem erfassten Akkommodationszustand (2) ein Steuersignal (79) für das medizinische Instrument (200) und/oder die medizinische Bildgebungseinrichtung (300) zu erzeugen und auszugeben.

2. Vorrichtung (100) nach Anspruch 1,
wobei die Ausgabeeinrichtung (120) dazu eingerichtet ist, das Steuersignal derart zu erzeugen, dass es Positionen, Ausrichtungen, und/oder Bewegungen in drei orthogonalen Raumrichtungen angibt, und wobei die Position, Ausrichtung, und/oder Bewegung bezüglich einer ersten dieser drei orthogonalen Raumrichtungen, insbesondere einer Tiefendimension, auf dem erfassten Akkommodationszustand (2) basiert.

3. Vorrichtung (100) nach Anspruch 2,
außerdem umfassend eine Orientierungs-Erfassungseinrichtung (130), welche dazu eingerichtet ist, eine Orientierung einer Pupille (4) des menschlichen Auges (1) zu erfassen;
wobei Positionen, Ausrichtungen, und/oder Bewegungen in einer zweiten sowie einer dritten der drei orthogonalen Raumrichtungen auf der erfassten Orientierung basieren.

4. Vorrichtung nach einem der Ansprüche 1 bis 3,
wobei die Akkommodations-Erfassungseinrichtung (110) eine Strahlungsquelle (111), eine Strahlungs-Erfassungseinheit (112), und eine Recheneinheit (113) aufweist,
wobei die Strahlungsquelle (111) dazu eingerichtet ist, eine Mess-Strahlung (71) in das menschliche Auge (1) auszusenden,
wobei die Strahlungs-Erfassungseinheit (112) dazu eingerichtet ist, eine von dem menschlichen Auge (1) zurückgeworfene, auf der ausgesendeten Mess-Strahlung (71) basierende Antwort-Strahlung (72) zu erfassen, und
wobei die Recheneinheit (113) dazu ausgebildet ist, basierend auf der erfassten Antwort-Strahlung (72) den Akkommodationszustand (2) des menschlichen Auges (1) zu bestimmen.

5. Vorrichtung (100) nach Anspruch 4,
wobei die Strahlungsquelle (111) zum Aussenden der Mess-Strahlung (71) mit einer Wellenlänge von 600 Nanometern bis 800 Nanometern, insbesondere von 625 Nanometern bis 700 Nanometern, besonders bevorzugt von 650 Nanometern bis 675 Nanometern, ausgebildet ist.

6. Vorrichtung (100) nach Anspruch 4 oder 5,
wobei die Strahlungsquelle (111) zum Aussenden der Mess-Strahlung (71) einen punktförmigen, linienförmigen oder kreuzförmigen Strahlungsemitter aufweist.

7. Vorrichtung (100) nach einem der Ansprüche 4 bis 6,
wobei die Vorrichtung (100) eine Datenbrille aufweist und zumindest die Strahlungsquelle (111) und/oder die Strahlungs-Erfassungseinheit (112) an der Datenbrille ausgebildet ist.

8. Vorrichtung (100) nach Anspruch 7,
wobei die Strahlungsquelle (111) dazu eingerichtet ist, die Mess-Strahlung (71) exzentrisch in das menschliche Auge (1) einzustrahlen.

9. Vorrichtung (100) nach einem der Ansprüche 4 bis 6,
wobei die Strahlungsquelle (111) und/oder die Strahlungs-Erfassungseinheit (112) zur stationären Montage in einem Raum eingerichtet ist.

10. Vorrichtung (100) nach einem der Ansprüche 1 bis 9,
wobei das Steuersignal (79) dazu ausgebildet ist, eine Bildselektion, eine Fokusselektion, und/oder eine Motivselektion in einer 3D-Darstellung einzustellen.

11. Vorrichtung (100) nach einem der Ansprüche 1 bis 10,
wobei das Steuersignal (79) dazu ausgebildet ist, eine Positions- oder Objektselektion in einer Virtuelle-Realität-Umgebung oder einer Erweiterte-Realität-Umgebung durchzuführen.

12. Verfahren zum Erzeugen eines Steuersignals (79) für ein medizinisches Instrument (200) und/oder eine medizinische Bildgebungseinrichtung (300), umfassend:
Erfassen (S10) eines Akkommodationszustands (2) eines menschlichen Auges (1); und
Erzeugen und Ausgeben (S20) eines Steuersignals (79) für ein medizinisches Instrument (200) oder eine medizinische Bildgebungseinrichtung (300) basierend auf dem erfassten Akkommodationszustand (2).

13. Verfahren nach Anspruch 12,
wobei das Erfassen (S10) des Akkommodationszustands (2) zumindest umfasst:
Erzeugen (S11) einer Mess-Strahlung (71);
Aussenden (S12) der Mess-Strahlung (71) in das menschliche Auge (1);
Erfassen (S13) einer auf der ausgesendeten Mess-Strahlung (71) basierenden, zurückgeworfenen Antwort-Strahlung (72); und
Bestimmen (S14) des Akkommodationszustands (2) basierend auf der erfassten Antwort-Strahlung (72).

14. Computerprogrammprodukt (400), umfassend ausführbaren Programmcode (450), welcher dazu eingerichtet ist, wenn er ausgeführt wird, das Verfahren gemäß Anspruch 12 oder Anspruch 13 auszuführen oder zu steuern.

15. Nicht-flüchtiges, computerlesbares Datenspeichermedium (500), umfassend ausführbaren Programmcode (550), welcher dazu eingerichtet ist, wenn er ausgeführt wird, das Verfahren gemäß Anspruch 12 oder Anspruch 13 auszuführen oder zu steuern.
